# EUROPEAN PATENT APPLICATION

(11) **EP 4 131 274 A1**
(43) Date of publication of application: **08.02.2023**
(21) Application number: 21190233.3
(22) Date of filing: 07.08.2021
(51) Int. Cl.: G16B 30/00, G16B 40/10, G16B 40/20

(54) **METHOD FOR CHARACTERIZATION OF CANCER**

(71) Applicant: Deutsches Krebsforschungszentrum - Stiftung des öffentlichen Rechts / Universität Heidelberg, 69120 Heidelberg (DE)
(72) Inventor: Sahm, Felix, 69120 Heidelberg (DE); Patel, Areeba, 69120 Heidelberg (DE); Dogan, Helin, 69120 Heidelberg (DE); Sill, Martin, 69120 Heidelberg (DE); Jones, David, 69120 Heidelberg (DE); Pfister, Stefan, 69120 Heidelberg (DE); von Deimling, Andreas, 69120 Heidelberg (DE); Loose, Matt, Nottingham (GB)
(74) Representative: Legl, Stefan

(57) **Abstract**

The present disclosure relates to a computer-implemented method for cancer diagnosis, comprising:
a) selectively sequencing polymers of a biological sample according to at least one target gene site by translocating the polymers through nanopores of a nanopore sequencing system, including:
(i) analyzing an initial nucleotide sequence of a first polymer of the biological sample while the first polymer is translocating through a nanopore of the nanopore sequencing system to determine whether the initial nucleotide sequence corresponds to the at least one target gene site; and
(ii) continuing the sequencing of the first polymer to obtain measurement data of the first polymer only if the initial nucleotide sequence of the first polymer corresponds to the at least one target gene site;

b) determining, based on the measurement data, a biological state of a nucleotide sequence of the first polymer corresponding to the at least one target gene site; and
c) classifying a cancer using a classification algorithm based on the biological state of the nucleotide sequence of the first polymer, wherein the classification algorithm is trained based on the at least one target gene site and biological state data pertaining to cancer types.

## Description

### FIELD

The present disclosure relates to the characterization of cancer, and more particularly to a computer-implemented method for classification and/or molecular characterization of cancer in diagnostic settings. The disclosure provides a method for characterizing a tumor sample obtained from a patient by analyzing a biological state, such as a methylation state, mutation or copy-number state, or mutational state, of selected gene sites using targeted nanopore sequencing. Embodiments of the present disclosure are particularly useful for characterization of various tumors, such as central nervous system tumors and/or sarcomas, as well as for identification of targets for therapeutic intervention. The term "characterization" as used throughout the present disclosure includes one or more of classification, diagnosis, treatment response prediction, and stratification.

### BACKGROUND

Molecular markers can be used for cancer diagnostics, such as brain tumor diagnostics. For example, DNA methylation-based classification of cancer provides a comprehensive molecular approach to diagnose tumors of the central nervous system (CNS). In fact, DNA methylation profiling of human brain tumors already profoundly impacts clinical neuro-oncology. This is complemented by the assessment of mutation, gene fusion and DNA copy-number status of important driver genes.

Nowadays multiple gene sequencing approaches are available for cancer diagnostics. However, conventional sequencing setups for complete molecular profiling require considerable investment, while batching samples for sequencing and methylation profiling can delay turnaround time.

Furthermore, neuropathology labs cannot rely on off-the-shelf products, since these do not cover the genes relevant for neuro-oncology. Thus, custom assays are typically set-up, provided the equipment for next-generation-sequencing (NGS) is available. In turn, advantages of custom neuropathology NGS panels can only be efficiently exploited when case numbers are sufficient. Labs with lower specimen submission numbers hence must pool samples over multiple weeks.

In light of the above, more advanced diagnostic tools are needed.

### SUMMARY

It is an object of the present disclosure to provide diagnostic techniques which are highly flexible, run efficiently on single samples, and/or can be initiated immediately upon receipt of, for example, frozen sections, liquid biopsy samples or cells from the biological sample. It is another object of the present disclosure to increase the speed of analysis of tumor samples, such as frozen section analysis.

The objects are solved by the features of the independent claims. Preferred embodiments are defined in the dependent claims. Any "aspect", "example" and "embodiment" of the description not falling within the scope of the claims does not form part of the invention and is provided for illustrative purposes only.

According to an independent aspect of the present disclosure, a computer-implemented method for cancer diagnosis is provided. The method includes:
a) selectively sequencing polymers of a biological sample according to at least one target gene site by translocating the polymers through nanopores of a nanopore sequencing system, including:
   (i) analyzing an initial nucleotide sequence of a first polymer of the biological sample while the first polymer is translocating through a nanopore of the nanopore sequencing system to determine whether the initial nucleotide sequence corresponds to the at least one target gene site; and
   (ii) continuing the sequencing of the first polymer to obtain measurement data of the first polymer only if the initial nucleotide sequence of the first polymer corresponds to the at least one target gene site;
b) determining, based on the measurement data, a biological state of a nucleotide sequence of the first polymer corresponding to the at least one target gene site; and
c) classifying a cancer using a classification algorithm based on the biological state of the nucleotide sequence of the first polymer, wherein the classification algorithm is trained based on the at least one target gene site and biological state data pertaining to cancer types.

The embodiments of the present disclosure combine targeted nanopore sequencing and a classification algorithm. Both the classification algorithm and the nanopore sequencing system use the same target gene sites, i.e., the nanopore sequencing system sequences only the target gene sites required by the classification algorithm to classify the cancer. In other words, the nanopore sequencing system selectively sequences certain polymers in a pool while rejecting other polymers. Therefore, the cancer classification of the present disclosure is very flexible in target selection, runs efficiently on single samples, and can be initiated immediately upon receipt of the biological sample, such as frozen sections, liquid biopsy samples, cells or FFPE (formalin-fixed paraffin-embedded tissue) samples.

In addition, the targeted nanopore sequencing of the present disclosure analyzes the initial nucleotide sequence, i.e., nucleotide data rather than raw measurement signals, to determine whether the initial nucleotide sequence corresponds to the at least one target gene site. Using the nucleotide sequence rather than raw signals (e.g., ionic current signals) to match the initial nucleotide sequence to the at least one target gene site utilizes reasonable computational resources and provides enrichment of targets. Furthermore, since the embodiments of the present disclosure do not use raw signal comparison to determine whether the initial nucleotide sequence corresponds to the at least one target gene site, there is no need to convert the reference genomes, i.e., the at least one target gene site, into signal space.

It should be understood that although the above was described using only the first polymer of the biological sample for clarity, hundreds, thousands, or even tens of thousands of polymers can be sequenced and used by the method of the present disclosure to classify the cancer type.

Nanopore sequencing is a third generation approach used in the sequencing of polymers, such as polynucleotides in the form of DNA or RNA. Using nanopore sequencing, a single molecule of DNA or RNA can be sequenced without the need for PCR amplification or chemical labeling of the sample. Thereby, nanopore sequencing offers low-cost sequencing, high mobility for testing, and rapid processing of samples with the ability to display results in real-time.

A nanopore sequencing system includes a biological or solid-state membrane on which one or more nanopores are located. The membrane is surrounded by electrolyte solution and splits the electrolyte solution into two chambers. When a bias voltage is applied across the membrane, an electric field is induced that puts charged particles, such as the ions of the electrolyte solution, into motion. Since the voltage drop concentrates near and inside the nanopore, charged particles experience a force from the electric field when the charged particles are near the pore region ("capture region"). Inside the capture region, motion of the ions provides a steady ionic current which can be measured by electrodes located at the membrane.

A polymer, such as DNA, also has a net charge that experiences a force from the electric field. Once inside the nanopore, the polymer translocates through the nanopore via electro-phoretic, electro-osmotic, and thermo-phoretic forces. Inside the nanopore the polymer partially restricts the ion flow, leading to a drop in the ionic current. Based on various factors such as geometry, size and chemical composition, the change in magnitude of the ionic current and the duration of the translocation varies. Different polymers can then be sensed and identified based on this modulation in ionic current.

According to some embodiments, which can be combined with other embodiments described herein, the nanopore and its corresponding electrodes constitute a sensor element. In some embodiments, the nanopore sequencing system includes an array of sensor elements. The array of sensor elements can be configured to take successive measurements of polymers from sensor elements selected in a multiplexed manner.

The embodiments of the present disclosure use selective sequencing. Selective sequencing refers to the ability of the nanopore sequencing system to decide whether a polymer should be fully sequenced or not while it is being sequenced. This requires a rapid classification of a current measurement signal from a first part of the read (i.e., the initial nucleotide sequence) to determine whether the polymer should be entirely sequenced or removed and replaced with a new polymer.

The decision whether a polymer should be fully sequenced or not is made based on a comparison between the initial nucleotide sequence of the first polymer and a reference genome, i.e., the at least one target gene site. In particular, the initial nucleotide sequence of the first polymer of the biological sample is analyzed while the first polymer is translocating through the nanopore to determine whether the initial nucleotide sequence corresponds to the at least one target gene site. Only if the initial nucleotide sequence of the first polymer matches a corresponding nucleotide sequence of a target gene site, the sequencing of the first polymer is continued.

An exemplary software module which can be used to implement the targeted sequencing is ReadFish. ReadFish is an open source software which enables targeted nanopore sequencing of gigabase-sized genomes. At least one target gene site can be included in the ReadFish-based targets to enable the targeted sequencing.

In the embodiments of the present disclosure, ReadFish works with nucleotide data rather than raw measurement signals to determine whether the initial nucleotide sequence corresponds to the at least one target gene site.

According to some embodiments, which can be combined with other embodiments described herein, the initial nucleotide sequence of the first polymer is a subset of the nucleotide sequence of the first polymer obtained when the first polymer has partially translocated through the nanopore. In particular, the subset of the nucleotide sequence is located at a beginning or front of the first polymer, wherein "beginning" and "front" refer to the leading end of the polymer that first enters the nanopore.

In typical applications, the decision whether a polymer should be fully sequenced or not can be made with sufficient accuracy after measurement of a few hundred nucleotides of the polymer, such as 500 nucleotides or less, 300 nucleotides or less, or even 100 nucleotides or less. This compares to the nanopore sequencing system being able to perform measurements on sequences ranging in length from several hundreds to tens of thousands of nucleotides.

In the embodiments of the present disclosure, only if the initial nucleotide sequence of the first polymer matches a corresponding nucleotide sequence of a target gene site, the sequencing of the first polymer is continued. However, if the initial nucleotide sequence of the first polymer does not correspond to (or matches a corresponding nucleotide sequence of) the at least one target gene site, the first polymer is rejected.

The rejection of the first polymer allows measurements of a further (second) polymer to be taken without completing the measurement of the first polymer. This provides a time saving in taking the measurements, because the action is taken during the taking of measurements from the first polymer. In particular, the analysis may identify at an early stage in such a read that no further measurements of the polymer currently being measured are needed.

The rejection of the first polymer may occur in different ways.

In a first example, rejecting the first polymer includes ejecting the first polymer from the nanopore. For example, the voltage across the nanopore can be reversed to eject the first polymer from the nanopore. Subsequently, the voltage across the nanopore can be reset to accept the second polymer in the nanopore.

In a second example, rejecting the first polymer includes ceasing taking measurements from the nanopore in which the first polymer is located. For example, the nanopore sequencing system includes an array of sensor elements. The array of sensor elements can be configured to take successive measurements of polymers from sensor elements selected in a multiplexed manner. In that case, the step of rejecting the first polymer may include to cease taking measurements from a currently selected sensor element and to start taking measurements from a newly selected sensor element.

The targeted nanopore sequencing of the present disclosure analyzes the initial nucleotide sequence, i.e., nucleotide data rather than raw measurement signals, to determine whether the initial nucleotide sequence corresponds to the at least one target gene site. Accordingly, the method may include a step of determining the initial nucleotide sequence from raw measurement data, such as ionic current data measured by the nanopore sequencing system described above.

In some embodiments, the step of determining the initial nucleotide sequence from raw measurement data may include: obtaining raw measurement data, such as ionic current data, from the nanopore; and performing (real-time) base-calling to obtain at least the initial nucleotide sequence of the first polymer.

According to some embodiments, which can be combined with other embodiments described herein, the further raw measurement data of the first polymer obtained when the initial nucleotide sequence of the first polymer corresponds to the at least one target gene site may also be processed using base-calling to determine the nucleotide sequence of the first polymer for which the biological state is determined in order to classify the cancer.

The term "base-calling" as used throughout the present disclosure refers to the process of assigning nucleobases to electrical current changes resulting from nucleotides passing through the nanopore.

According to some embodiments, which can be combined with other embodiments described herein, the base-calling uses a neural network, in particular a recurrent neural network (RNN).

Real-time base-calling software is available e.g. from Oxford Nanopore Technologies (ONT). ONT have developed a number of base-callers for nanopore sequence data, initially utilizing hidden Markov models and available through the metrichor cloud service. They replaced these with neural network models running on central processing units and then GPUs. For real-time base-calling, ONT provide a range of computational platforms with integrated GPUs (minIT, Mk1C, GridION and PromethION). These devices enable real-time base-calling sufficient to keep pace with nanopores generating data. Most recently, these base-callers acquired a server-client configuration, such that raw signal can be passed to the server and a nucleotide sequence returned.

The embodiments of the present disclosure may use GPU base-calling to deliver a real-time stream of nucleotide data from nanopore sequencing e.g. with up to 512 channels simultaneously. At the same time, the GPU can base-call completed reads, and optimized tools such as minimap can therefore be used to map reads as they are generated, enabling dynamic updating of both the targets and the reference genome as results change.

According to some embodiments, which can be combined with other embodiments described herein, selectively sequencing polymers of a biological sample according to at least one target gene site of step a) includes: sequencing (i) the nucleotides of the first polymer corresponding to the at least one target gene site and (ii) a predetermined number of nucleotides upstream and/or downstream of the at least one target gene site. Accordingly, one or two flanks can be added to (a) target gene site(s) to ensure optimal targeting.

According to some embodiments, which can be combined with other embodiments described herein, the predetermined number of nucleotides upstream and/or downstream of the at least one target gene site is 25 kb or up to 25 kb. In some embodiments, the predetermined number of nucleotides upstream and/or downstream of the at least one target gene site is 10 kb or up to 10 kb, or preferably 20 kb or up to 20 kb, or preferably 30 kb or up to 30 kb, or preferably 50 kb or up to 50 kb, or preferably 75 kb or up to 75 kb, or preferably 100 kb or up to 100 kb. Preferably, a 10kb upstream and 10kb downstream flank is provided for all target gene sites.

According to some embodiments, which can be combined with other embodiments described herein, the biological state used to classify the cancer is selected from the group including (or consisting of) a mutation state and an epigenetic state of the nucleotide sequence of the first polymer.

The biological state can be derived from the measurement data, such as the raw measurement data or ionic current data, obtained by the nanopore sequencing system. In other words, the raw measurement data, such as the ionic current data, may provide information regarding the nucleotide sequence and the biological state.

According to some embodiments, which can be combined with other embodiments described herein, the biological state can be derived from the nucleotide sequence and/or the raw measurement data using one or more neural networks, in particular deep learning-based neural networks.

The mutation state may be a copy number variation (CNV). Copy number variation is a phenomenon in which sections of the genome are repeated and the number of repeats in the genome varies between individuals. CNV can be derived directly from the nucleotide sequence obtained by base calling.

The term "epigenetic state" refers to a measure for epigenetic changes (or for functionally relevant changes of an upregulation and/or downregulation) of the gene activity of a particular gene site and/or gene in the genome of a biological sample. The epigenetic state comprises an epigenetic downregulation and/or upregulation of the gene site's activity in the biological sample in comparison to that same gene site's activity in physiological tissue. Such downregulation and/or upregulation can for example be due to DNA methylation, histone modification or other epigenetic effects.

The epigenetic state may be a methylation state. Nanopore sequencing enables to directly detect methylation states of bases in DNA from reads without extra laboratory techniques. Methylation states may be determined by suitable software modules, such as megalodon by Oxford Nanopore Technologies (ONT). Megalodon is a research command line tool to extract high accuracy modified base and sequence variant calls from raw nanopore reads by anchoring the information rich base-calling neural network output to a reference genome/transriptome. Megalodon is publicly available at https://github.com/nanoporetech/megalodon.

The term "methylation state", as used herein may describe the state of methylation of a CpG position, thus may refer to the presence or absence of 5-methylcytosine at one CpG site within genomic DNA. When none of the DNA of an individual is methylated at one given CpG site, the position is 0% methylated. When all the DNA of the individual is methylated at that given CpG site, the position is 100% methylated. When only a portion, e.g., 50%, 75%, or 80%, of the DNA of the individual is methylated at that CpG site, then the CpG position is said to be 50%, 75%, or 80%, methylated, respectively.

The term "methylation state" reflects any relative or absolute amount of methylation of a gene site, in particular a CpG position. The terms "methylation" and "hypermethylation" are used herein interchangeably. When used in reference to a CpG positions, they refer to the methylation state corresponding to an increased presence of 5-methylcytosine at a CpG site within the DNA of a biological sample obtained from a patient, relative to the amount of 5-methylcytosine found at the CpG site within the same genomic position of a biological sample obtained from a healthy individual, or alternatively from an individual suffering from a tumor of a different class or species.

According to some embodiments, which can be combined with other embodiments described herein, the at least one target gene site corresponds to at least one CpG position.

As used herein, the term "CpG site" or "CpG position" refers to a region of DNA where a cytosine nucleotide occurs next to a guanine nucleotide in the linear sequence of bases along its length, the cytosine (C) being separated by only one phosphate (p) from the guanine (G). About 70% of human gene promoters have a high CpG content. Regions of the genome that have a higher concentration of CpG sites are known as "CpG islands". Cytosines in CpG dinucleotides can be methylated to form 5-methylcytosine. Methylation of (i.e., introduction of a methyl group in) the cytosines of CpG site within the promoters of genes can lead to gene silencing, a feature found in a number of human cancers. In contrast, the hypomethylation of CpG sites has generally been associated with the overexpression of oncogenes within cancer cells. The term "independent genomic CpG positions" shall in the context of the present disclosure mean that each CpG position of a group of genomic CpG positions can be probed separately for its methylation status.

According to some embodiments, which can be combined with other embodiments described herein, the at least one target gene site is a plurality (or set) of target gene sites, and wherein a respective biological state is determined for each target gene site of the plurality (or set) of target gene sites.

Preferably, the set of target gene sites comprises at least 10, preferably at least 20 or at least 30 or at least 40 or at least 50 or at least 60 or at least 70 or at least 80 or at least 90 or at least 100 target gene sites. In some embodiments, a set of target gene sites can be defined which can be used to characterize a plurality of cancer types. In other words, the same set of target gene sites can be used to characterize different cancer types.

As used herein, the term "gene site" refers to a region of DNA comprising or consisting of a gene, particularly a gene or gene site, suitable to identify a cancer type. In particular, the term "gene site" refers to a DNA sequence with a genetic locus. A gene site may comprise additional base pairs upstream and/or downstream of a gene, for example up to 12 kb, preferably up to 10 kb up to 8 kb or up to 6 kb or up to 4 kb or up to 2 kb upstream and/or downstream of the gene. A biological state of a gene site may therefore refer to the biological state of the gene itself and additionally to the biological state of the additional string of base pairs upstream and/or downstream of the gene.

In one embodiment of the disclosure, DNA methylation can be used to find gene sites with pathological activity within a cancer genome. Thus, a set of gene sites having the biggest impact on differentiating between different cancer types can be defined and used for both the targeted nanopore sequencing and the cancer classification.

Once the biological state(s) of the target gene site(s) has/have been determined, the cancer type of the biological sample can be determined using the classification algorithm. The classification algorithm is trained based on the at least one target gene site and biological state data pertaining to different cancer types. Accordingly, the embodiments of the present disclosure provide a classification of biological samples in cancer diagnosis using a classification algorithm, which can be a machine learning (ML) algorithm.

The term "classification" refers to a procedure and/or algorithm in which individual items are placed into groups or classes based on quantitative information on one or more characteristics inherent in the items (referred to as traits, variables, characters, features, etc.) and based on a statistical model and/or a training set of previously labelled items. Specifically in the context of the present disclosure, classification preferably means determining which specific cancer type, for example determined by its epigenetic features, a biological sample belongs to.

The term "machine learning algorithm" as used throughout the present application refers to an algorithm that builds a model based on training data, in order to make predictions or decisions without being explicitly programmed to do so. In particular, the term "classification" refers to a machine learning algorithm in which individual items are placed into groups or classes based on quantitative information on one or more characteristics inherent in the items (referred to as traits, variables, characters, features, etc.) and based on a statistical model and/or a training data set of previously labelled items. Specifically in the context of the present invention, classification preferably means determining which specific cancer type, for example determined by its epigenetic status pattern, a biological sample belongs to.

The term "training data set" in context of the disclosure refers to a set of biological state data, such as genomic methylation data, of a multitude of tumors that were classified by prior art methods, and therefore are of known tumor species.

The classification algorithm can be any appropriate algorithm for establishing a correlation between datasets, namely the biological state of the DNA polymers of the biological sample and the biological state data derived from pre-classified cancer types. Methods for establishing correlation between datasets include, but are not limited to, discriminant analysis (DA) (e.g., linear-, quadratic-, regularized-DA), Discriminant Functional Analysis (DFA), Kernel Methods (e.g., SVM), Nonparametric Methods (e.g., k-Nearest-Neighbor Classifiers), PLS (Partial Least Squares), Tree-Based Methods (e.g., CART, Random Forest Methods), Gradient Boosting, Generalized Linear Models (e.g., Logistic Regression), Principal Components based Methods (e.g., SIMCA), Generalized Additive Models, Fuzzy Logic based Methods, Neural Networks and Genetic Algorithms based Methods.

The person skilled on the art will have no problem in selecting an appropriate method/algorithm to establish the correlation between the biological state data of the biological sample and the biological state data derived from pre-classified cancer types.

In an exemplary embodiment, the classification algorithm uses random forest analysis. As used herein the term "random forest analysis" refers to a computational method that is based on the idea of using multiple different decision trees to compute the overall most predicted class (the mode). In a specific application, the mode will be either tumor species or class based on how many decision trees predicted the samples to match a specific class. The class predicted by the majority is selected as the predicted class for the sample. The different decision trees used in this algorithm are trained on a randomly generated subset of the training data set and on a randomly selected set of the variables.

Examples for the correlation between target gene sites and cancer types as well as suitable classification algorithms are provided in EP 3 268 492 B1and Capper, D., Jones, D., Sill, M. et al.; DNA methylation-based classification of central nervous system tumours; Nature 555, 469-474 (2018); https://doi.org/10.1038/nature26000.

The embodiments of the present disclosure allow for the characterization of cancer. The term "characterization" as used throughout the present disclosure includes one or more of classification, diagnosis, and stratification.

The term "diagnosis" is used herein to refer to the identification or classification of a molecular or pathological state, disease or condition. For example, "diagnosis" may refer to identification of a particular type of cancer, e.g., of the central nervous system (CNS). It is important to note that the disclosure is directed to a strictly in vitro method in all its embodiments. None of the method steps of any embodiment are performed on the human or animal body.

The term "stratification" refers to the classification or grouping of patients according to one or more predetermined criteria. In certain embodiments stratification is performed in a diagnostic setting in order to group a patient according to the prognosis of disease progression, either with or without treatment. In particular embodiments stratification is used in order to distribute patients enrolled for a clinical study according to their individual characteristics. Stratification may be used in order to identify the best suitable treatment option for a patient.

The term "sample" or "biological sample" is used herein in its broadest sense. In the practice of the present disclosure, a biological sample is generally obtained from a subject. A sample may be any biological tissue or fluid with which the biological state of the present disclosure may be assayed. Frequently, a sample will be a "clinical sample" (i.e., a sample obtained or derived from a patient to be tested). The sample may also be an archival sample with known diagnosis, treatment, and/or outcome history.

Examples of biological samples suitable for use in the practice of the present disclosure include, but are not limited to, bodily fluids, e.g., blood samples (e.g., blood smears), and cerebrospinal fluid, brain tissue samples, spinal cord tissue samples or bone marrow tissue samples such as tissue or fine needle biopsy samples. Biological samples may include preserved samples, such as e.g. samples preserved in alcohol or Formalin-Fixed Paraffin-Embedded (FFPE) tissue samples. Biological samples may also include sections of tissues such as frozen sections taken for histological purposes. The term "biological sample" also encompasses any material derived by processing a biological sample. Derived materials include, but are not limited to, cells (or their progeny) isolated from the sample, as well as nucleic acid molecules (DNA and/or RNA) extracted from the sample. Processing of a biological sample may involve one or more of: filtration, distillation, extraction, concentration, inactivation of interfering components, addition of reagents, and the like.

Preferably, the biological sample is a cancer sample.

The term "cancer" or "tumor" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies etc. are included.

The term "cancer sample" or "tumor sample" as used herein refers to a sample obtained from a patient. The tumor sample can be obtained from the patient by routine measures known to the person skilled in the art, i.e., biopsy (taken by aspiration or punctuation, excision or by any other surgical method leading to biopsy or resected cellular material). For those areas not easily reached via an open biopsy a surgeon can, through a small hole made in the skull, use stereotaxic instrumentation to obtain a "closed" biopsy. Stereotaxic instrumentation allows the surgeon to precisely position a biopsy probe in three-dimensional space to allow access almost anywhere in the brain. Therefore, it is possible to obtain tissue for the diagnostic method of the present disclosure. The actual removal of the sample from the patient is, however, not part of the inventive method. "Providing a cancer sample" therefore merely pertains to making a sample available for laboratory use without the step of obtaining it from a patient in the first place.

In some embodiments, a biological sample includes, but is not limited to, biological fluids comprising biomarkers, cells, tissues, and cell lines. In some embodiments, a biological sample includes, but is not limited to, primary cells, induced pluripotent cells (IPCs), hybridomas, recombinant cells, whole blood, stem cells, cancer cells, bone cells, cartilage cells, nerve cells, glial cells, epithelial cells, skin cells, scalp cells, lung cells, mucosal cells, muscle cells, skeletal muscles cells, striated muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymuscells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cell types thereof.

According to another independent aspect of the present disclosure, a computer-readable storage medium is provided. The computer-readable storage medium has computer-executable instructions stored, that, when executed, cause a computer to perform the methods described herein.

The term "computer-readable storage medium" may refer to any storage device used for storing data accessible by a computer, as well as any other means for providing access to data by a computer. Examples of a storage device-type computer-readable medium include: a magnetic hard disk; a floppy disk; an optical disk, such as a CD-ROM and a DVD; a magnetic tape; a memory chip.

According to another independent aspect of the present disclosure, a system for diagnosing cancer is provided. The system includes one or more processors and a memory coupled to the one or more processors and comprising instructions executable by the one or more processors to implement the methods described herein.

The system may be a computer system. The term a "computer system" may refer to a system having a computer, where the computer comprises a computer-readable storage medium embodying software to operate the computer.

The term "software" is used interchangeably herein with "program" and refers to prescribed rules to operate a computer. Examples of software include: software; code segments; instructions; computer programs; and programmed logic.

Further aspects, benefits, and features of the present disclosure are apparent from the claims, the description, and the accompanying drawings.

### BRIEF DESCRIPTION OF THE DRAWINGS

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1: shows a flow chart of a computer-implemented method for cancer diagnosis according to embodiments described herein;
- FIG. 2A: shows a timeline for conventional NGS panel sequencing and analysis pipeline, and a timeline for conventional EPIC array analysis pipeline for neuropathology diagnostics;
- FIG. 2B: shows a timeline for RAPID-CNS sequencing and analysis pipeline for a single sample according to embodiments of the present disclosure;
- FIG. 3: shows a concordance of clinically relevant alterations and classification;
- FIG. 4: shows CNV plots obtained using RAPID-CNS, panel-sequencing, and EPIC array analysis; and
- FIG. 5: shows MGMT promoter methylation values averaged over the MGMT promoter region.

### DETAILED DESCRIPTION OF EMBODIMENTS

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

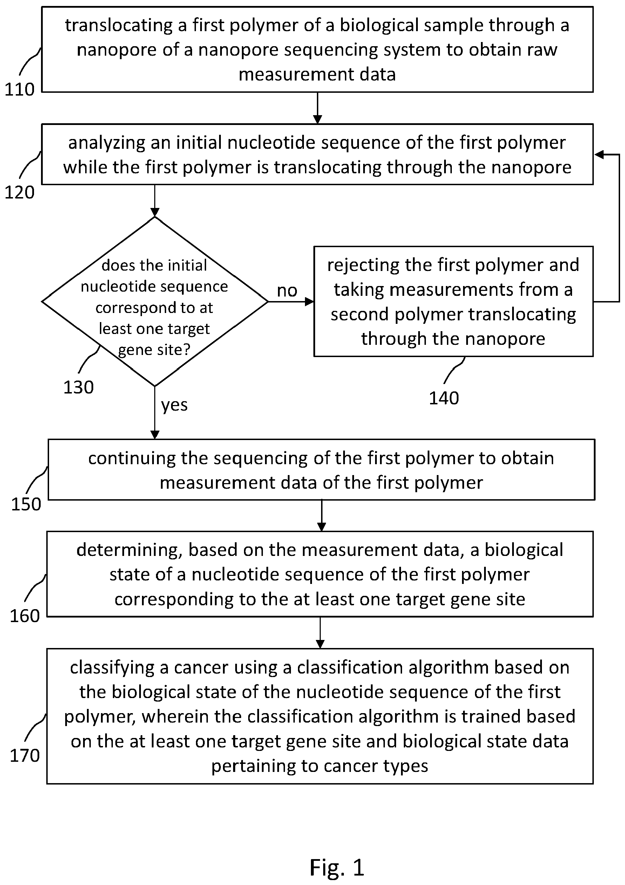

FIG. 1 shows a flowchart of a computer-implemented method 100 for cancer diagnosis, in particular rapid comprehensive adaptive nanopore-sequencing of CNS tumors (RAPID-CNS or RAPD-CNS²)

The method 100 combines targeted nanopore sequencing and a classification algorithm. Both the classification algorithm and the nanopore sequencing system use the same target gene sites, i.e., the nanopore sequencing system sequences only the target gene sites required by the classification algorithm to classify the cancer. In other words, the nanopore sequencing system selectively sequences certain polymers in a pool while rejecting other polymers. Therefore, the cancer classification of the present disclosure is very flexible in target selection, runs efficiently on single samples, and can be initiated immediately upon receipt of frozen sections.

In detail:
First, a biological sample is provided. The biological sample can be obtained from a patient by routine measures known to the person skilled in the art, such as biopsy. The actual removal of the biological sample from the patient is, however, not part of the inventive method. "Providing a biological sample" therefore merely pertains to making a sample available for laboratory use without the step of obtaining it from a patient in the first place.

A nanopore sequencing system is used to selectively sequence polymers of the biological sample. The selective sequencing is done using a reference genome, i.e., at least one target gene site. During the selective sequencing, a first polymer of the biological sample is translocated through a nanopore of the nanopore sequencing system to obtain raw measurement data (block 110).

An initial nucleotide sequence of the first polymer is derived from the raw measurement data using, for example, base-calling, and analyzed in block 120 while the first polymer has partially translocated through the nanopore. The initial nucleotide sequence of the first polymer may have a length of a few hundred nucleotides, such as 500 nucleotides or less, 300 nucleotides or less, or even 100 nucleotides or less.

In block 130 it is determined whether the initial nucleotide sequence corresponds to the at least one target gene site.

If it is determined that the initial nucleotide sequence does not correspond to the at least one target gene site, the first polymer first polymer is rejected to allow measurements of a further (second) polymer to be taken without completing the measurement of the first polymer (block 140).

If it is determined that the initial nucleotide sequence corresponds to the at least one target gene site, the method 100 proceeds with block 150 in which the sequencing of the first polymer is continued to obtain (raw) measurement data of the first polymer. Based on these measurement data of the first polymer, a nucleotide sequence of the first polymer can be determined. This nucleotide sequence of the first polymer may include the initial nucleotide sequence determined earlier.

In block 160, a biological state of the nucleotide sequence of the first polymer corresponding to the at least one target gene site is then determined based on the measurement data obtained in block 150.

Finally, in block 170, a cancer type of the biological sample is determined using a classification algorithm based on the biological state of the nucleotide sequence of the first polymer. The classification algorithm is trained based on the at least one target gene site and biological state data pertaining to cancer types.

It should be understood that although the above example was described using only the first polymer of the biological sample for clarity, hundreds, thousands, or even tens of thousands of polymers can be sequenced and used in block 170 to classify the cancer type.

### EXAMPLE: Glioma patient samples

### Brief Summary of the Example

The WHO classification 2021 includes multiple molecular markers for routine diagnostics in addition to histology. Sequencing setup for complete molecular profiling requires considerable investment, while batching samples for sequencing and methylation profiling can delay turnaround time. The present disclosure introduces RAPID-CNS, a nanopore adaptive sequencing pipeline that enables comprehensive mutational, methylation and copy number profiling of CNS tumors with a single third generation sequencing assay. It can be run for single samples and offers highly flexible target selection requiring no additional library preparation.

Utilizing ReadFish (https://github.com/LooseLab/readfish), a toolkit enabling targeted nanopore sequencing, the inventors sequenced DNA from 22 diffuse glioma patient samples on a MinION device (Oxford Nanopore Technologies Ltd). Target regions comprised the Heidelberg brain tumor NGS panel and pre-selected CpG sites for methylation classification by an adapted random forest classifier. Pathognomonic alterations, copy number profiles, and methylation classes were called using a custom bioinformatics pipeline. They were compared to their corresponding NGS panel sequencing and EPIC array results.

Complete concordance with the EPIC array was found for copy number profiles: 94% pathognomonic mutations were congruent with NGS panel-seq. MGMT promoter status was correctly identified in all samples. Methylation families were detected with 96% congruence. Among the alterations decisive for rendering a classification-compatible integrated diagnosis, 97% of the alterations were consistent over the entire cohort (completely congruent in 19/22 cases and sufficient for unequivocal diagnosis in all).

RAPID-CNS provides a swift and highly flexible alternative to conventional NGS and array-based methods for SNV/Indel analysis, detection of copy number alterations and methylation classification. The turnaround time of ~5 days can be further shortened to less than 24hrs by altering target sizes and enabling real-time analysis. It offers a low-capital approach that would be cost-efficient for low throughput settings and beneficial in cases requiring immediate diagnoses.

### Detailed Description of the Example

Molecular markers are decisive for brain tumor diagnostics. The WHO classification of brain tumors 2021 substantially increases the set of genes required in routine evaluation. Multiple sequencing approaches are available. However, neuropathology labs cannot rely on off-the-shelf products, since these do not cover the genes relevant for neuro-oncology, while including large target regions that are dispensable. Thus, custom assays are typically set-up, provided the equipment for next-generation-sequencing (NGS) is available. In turn, advantages of custom neuropathology NGS panels can only be efficiently exploited when case numbers are sufficient. Labs with lower specimen submission numbers hence must pool samples over multiple weeks.

The present document introduces RAPID-CNS - a custom neuro-oncology based workflow using third generation sequencing for copy-number profiling, mutational and methylation analysis that is highly flexible in target selection, runs efficiently on single samples, and can be initiated immediately upon receipt of frozen sections.

Nanopore sequencing has an advantage over current NGS methods in terms of longer read sizes, shorter and easier library preparation, ability to call base modifications, real time analysis, and portable sequencing devices, all at low costs. However, smaller devices like the MinION (Oxford Nanopore Technologies Ltd) yield low coverage data that makes it difficult to detect pathognomonic alterations or hard-to-map regions like the TERT promoter. Nanopore provides a "ReadUntil" adaptive sampling toolkit that can reject reads in real-time during sequencing. ReadFish harnesses this functionality to enable targeted adaptive sequencing with no additional steps in library preparation. This considerably increases coverage over "target" regions by enrichment during sequencing, which should allow confident detection of clinically relevant alterations.

RAPID-CNS can leverage adaptive nanopore sequencing through ReadFish and is run using a portable MinION device (Oxford Nanopore Technologies Ltd). The inventors formulated target regions covering the brain tumour NGS panel and CpG sites required for methylation classification and performed ReadFish based sequencing on 22 diffuse glioma samples. These previously underwent the brain tumor NGS panel and Infinium MethylationEPIC array (EPIC) analysis.

Samples were selected to cover pathognomonic alterations (IDH, 1p/19q codeletion, chr7 gain/chr10 loss, TERT promoter, EGFR amplification, CDKN2A/B deletion, MGMT status) and relevant methylation classes identified by conventional methods.

Cryo-conserved brain tumor tissue was prepared for nanopore sequencing with the SQK-LSK109 Ligation Sequencing Kit from ONT. Incubation time and other parameters were optimized to improve quality, amount of data generated and on-target rate of the libraries (see section "Supplementary Methods").

Single samples were loaded onto FLO-MIN106 R9.4.1 flow cells and run a MinION 1B (Oxford Nanopore Technologies Ltd). ReadFish controlled the sequencing in real-time and was run using a consumer notebook powered by an 8GB NVIDIA RTX 2080 Ti GPU. Samples were sequenced for up to 72 hours Our targets covered 5.56% of the entire genome. Sequencing time can be reduced to 24 hours by reducing the size of the targeted regions.

Sequenced data was analyzed using a bioinformatics pipeline customized for neuro-oncology targets. SNVs were filtered for clinical relevance by their 1000 genomes population frequency (<0.01) and COSMIC annotations. Copy number alterations were estimated using depth-of-coverage of the mapped reads.

Nanopore sequencing provides the additional advantage of estimating base modifications from a single DNA sequencing assay. Methylated bases were identified using megalodon, a deep neural network based modified base caller. Megalodon's output was used to compute methylation values over targeted CpG sites and assess MGMT promoter methylation status.

A random forest classifier based on the previously published reference set (Capper, D., Jones, D., Sill, M. et al.; DNA methylation-based classification of central nervous system tumours; Nature 555, 469-474 (2018); https://doi.org/10.1038/nature26000) was trained to predict methylation classes for the samples. MGMT promoter methylation status was assigned by averaging methylation values over all CpG sites in the MGMT promoter region (see section "Supplementary Results"). Mean run time from tissue collection to reporting for RAPID-CNS was less than 5 days.

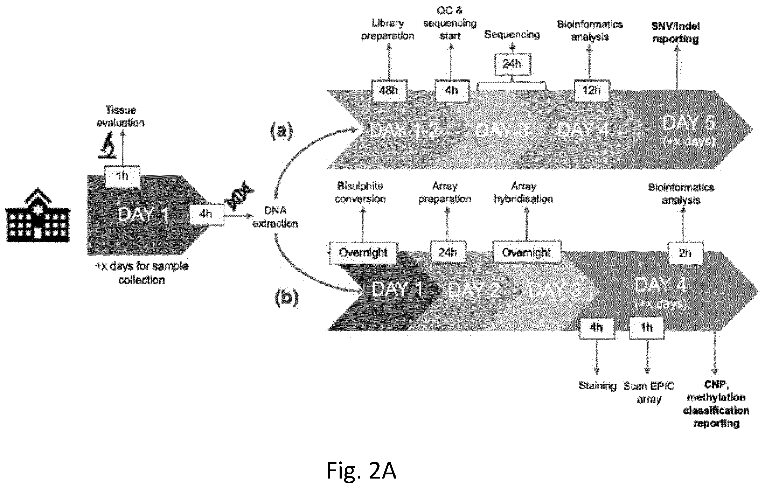

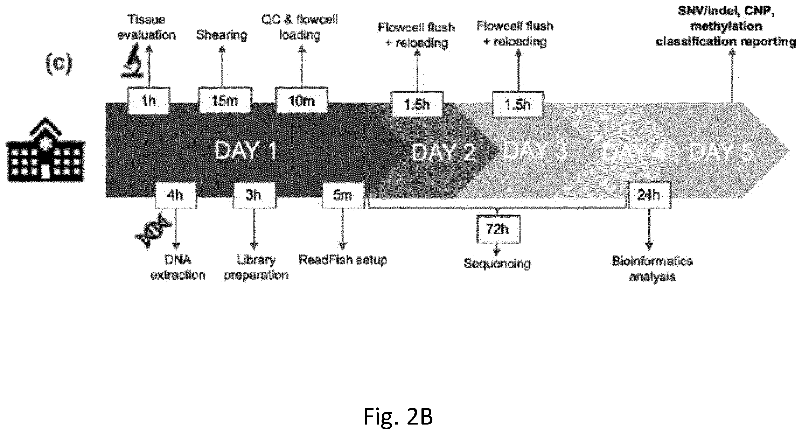

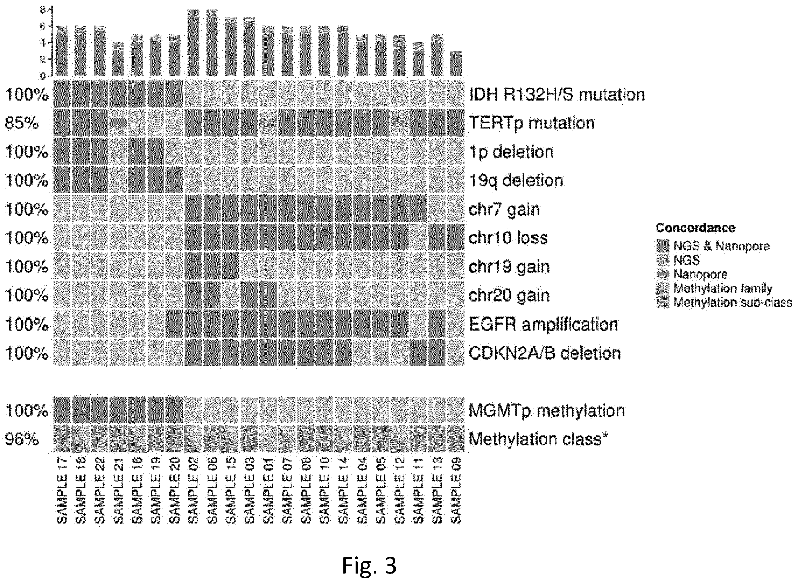

Nanopore sequencing considerably reduced library preparation time to 3.5 hours, as opposed to 48 hours for panel sequencing and 72 hours for the EPIC array (Figures 2A and 2B). Thereby, it merged both data categories into one lab workflow. Despite the differences in sequencing technology and method-specific data analysis pipelines, congruence of detected SNVs, regardless of clonality and clinical relevance, was 78%. Importantly, diagnostically relevant, pathognomonic mutations like IDH1 R132H/S and TERT promoter were congruent in 22/22 and 19/22 samples respectively (FIG. 3: coloured blocks indicate presence of alteration, concordance for detected alterations is denoted in the legend. Triangular denotations for methylation class indicate samples where methylation families were concordant, and blocks indicate concordance for sub-classes as well. Percentages on the left indicate concordance for the alteration over all samples).

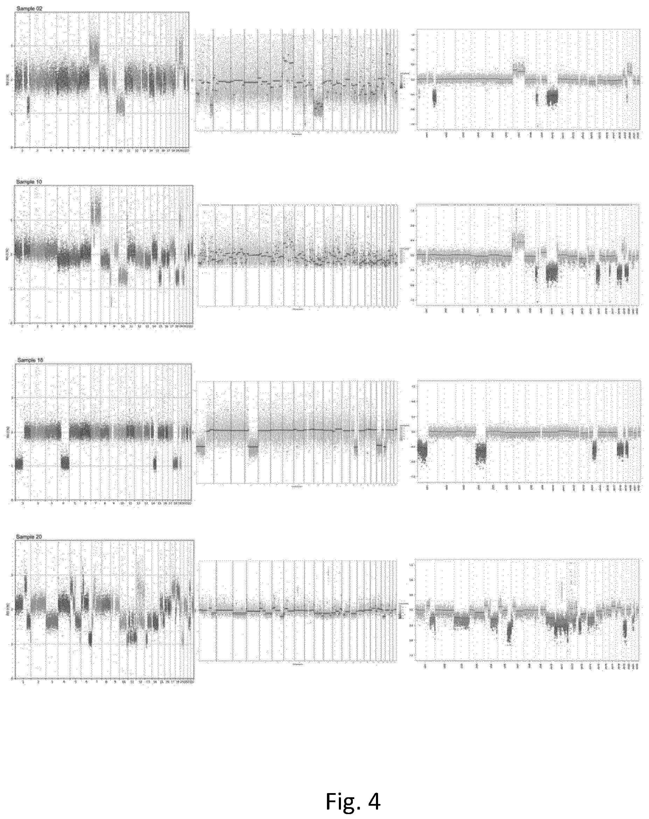

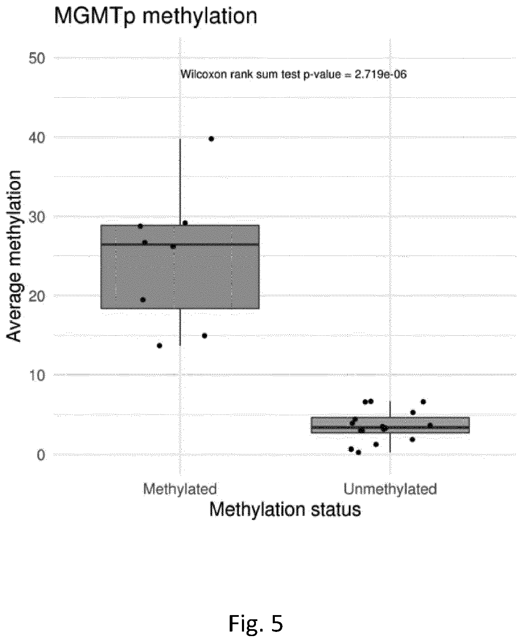

In addition, the inventors derived copy-number-plots (CNP) from calculated copy number levels for the Nanopore data (Supplementary figure 1a). Plots generated using nanopore data displayed markedly better resolution than those obtained using panel sequencing data (FIGs. 4 and 5). Complete concordance with EPIC array analysis was found for CNV levels in all samples. RAPID-CNS also enabled gene-level CNV detection. Among the alterations decisive for rendering an integrated molecular diagnosis, 217/220 were consistent over the entire cohort (completely congruent in 19/22 cases).

Including methylation-classification relevant CpGs in ReadFish-based targets also allowed for methylation class prediction. The ability of nanopore sequencing to reliably serve in methylation classification using low pass whole genome nanopore sequencing data has previously been demonstrated by nanoDx (https://www.medrxiv.org/content/10.1101/2021.03.06.21252627v1.full). Methylation families predicted by RAPID-CNS matched their corresponding EPIC array-based classification in 21/22 cases, while methylation sub-classes were concordant in 14 cases. MGMT promoter status was also congruent with its corresponding EPIC array analysis for all cases. Nanopore identified a MGMT promoter status as unmethylated in one sample in line with the EPIC array, which was assigned as methylated by pyrosequencing.

Targeted regions for RAPID-CNS can be easily altered by editing a BED file, in principle allowing lower sequencing times than in this study. With no additional library preparation steps required, it is possible to modify targeted regions for each sample as required. The MinION is a portable, handheld device which makes it a rational option for smaller neuropathology labs as well.

While the inventors used a GPU to run ReadFish, it can be run using a sufficiently powerful CPU as well. Collectively, the RAPID-CNS approach can be set-up at low capital expense, is cost-efficient even in a low throughput setting, and provides a swift and highly flexible alternative to conventional NGS methods for SNV/Indel analysis and, if corresponding methods e.g. methylation array are not run, also for methylation classification and detection of copy-number-alterations.

### Supplementary Methods

### 1. Nanopore library prep optimized for adaptive sampling

Sections of 40x10 µm were prepared from cryo-conserved tumor tissue with established molecular markers (IRB approval 2018-614N-MA, 005/2003) if tumor cell content (based on a H&E stain) was > 60%. DNA was then extracted using the Promega Maxwell RSC Blood DNA Kit (catalogue # AS 1400, Promega) on a Maxwell RSC 48 instrument (AS8500, Promega) per manufacturer's instructions. DNA concentrations were measured on a microplate reader (FLUOStar Omega, BMG Labtech) using the Invitrogen Qubit DNA BR Assay Kit (Q32851, Thermo Fisher Scientific).

Next, the DNA was sheared to approximately 9 to 11 kb in a total volume of 50 µl using g-TUBEs (Covaris) at 7200 rpm for 120 sec. The fragment length was assessed on an Agilent 2100 Bioanalyzer (catalogue # G2939A, Agilent Technologies) with the Agilent DNA 12000 Kit (catalogue # 5067-1508, Agilent Technologies).

Sequencing libraries were prepared with the SQK-LSK109 Ligation Sequencing Kit with the following modifications: 48 µl of the sheared DNA (2-2.5 µg) were taken into the end-prep reaction leaving out the control DNA. The end-prep reaction was changed to an incubation for 30min at 20° followed by 30min at 65° followed by a cool down to 4° in a thermal cycler. The clean-up was performed using AMPure XP beads and 80% ethanol, elution time was changed to 5min. Adapter ligation was extended to an incubation for 60min at room temperature. The ligation mix was then incubated with AMPure XP beads at 0.4x for 10min, clean-up was performed using the Long Fragment Buffer (LFB) and the final library was eluted in a total volume of 31 µl.

Library concentrations were measured using the Invitrogen Qubit DNA HS Assay Kit (Q32851, Thermo Fisher Scientific) on a benchtop Quantus fluorometer (Promega). The libraries were loaded (500-600 ng) onto FLO-MIN106 R9.4.1 flow cells with a minimum of 1100 pores available according to the FC Check prior to loading. The flow cells were flushed after around 24 hours for a total of two times per sample with the Flow Cell Wash Kit (EXP-WSH003) per manufacturer's instructions. All sequencing was carried out on a MinION 1B (Oxford Nanopore Technologies).

### 2. ReadFish

Targeted nanopore sequencing was performed in real-time using a custom panel with ReadFish on an 8 GB NVIDIA RTX 2080 Ti powered consumer notebook. The targets included regions from the neuropathology panel and CpG sites instrumental in classification by the random forest methylation classifier (available on GitHub). A 25kbp flank was added to the sites to ensure optimal targeting by ReadFish. Guppy 4.4.1's fast base-calling mode was used to run ReadFish.

### 3. Methylation classification

To classify nanopore sequencing derived DNA-methylation profiles of central nervous system tumors, a random forest classifier was trained on publicly available 450k methylation array reference data set of the MNP classifier version 11 (GSE90496). This data set was preprocessed as described in Capper, D., Jones, D., Sill, M. et al.; DNA methylation-based classification of central nervous system tumours; Nature 555, 469-474 (2018); https://doi.org/10.1038/nature26000.

For a batch of 22 nanopore sequencing samples, intersection of CpG probes measured for all samples were selected to train the classifier. The methylation array data set was reduced to these 3,285 probes.

Often nanopore sequencing measures CpG probes with low coverage, which leads to discrete distributed methylation values, i.e. (0, 0.5, 1). As finer methylation differences can often not be detected with nanopore sequencing for all CpG probes, the inventors trained the RF classifier on dichotomized methylation values. This followed the assumption that splitting rules learned on binary data are more robust and can be applied to methylation signals from nanopore sequencing data.

After dichotomizing the reduced reference methylation data set, a RF was trained with 1000 trees and the resulting permutation based variable importance measure was applied to select the 1000 CpGs with highest variable importance to train a final RF with again 1000 trees. The out-of-bag of this classifier was 4%.

### Supplementary Results

### 1. RAPID-CNS analysis pipeline

The bioinformatics pipeline requires raw FAST5 files as input. Complete instructions for setting up the analysis are available on GitHub. Post set-up, RAPID-CNS runs the entire analysis with a single command. It can be run on an LSF cluster or a GPU workstation. Base-calling followed by SNV and CNV detection completes within 10 hours while methylation calling and classification requires an additional 12 hours.

### 2. SNV detection

ANNOVAR annotated tables for all nanopore sequenced samples and their corresponding panel sequencing results are available to the inventors.

### 3. CNV detection

Copy number plots obtained using the RAPID-CNS pipeline demonstrate higher resolution and clear visualization of the copy number levels as compared to NGS panel sequencing (FIG. 4, left and center). Calculating depth of mapped reads, copy number variations detected are comparable to EPIC array results (FIG. 4, left and right). Normalized read depths are indicated on the Y-axis with "2" indicating mean autosomal level. Additionally, genes covered by the copy number variations and their zygosity are annotated and output as excel files (available to the inventors).

### 4. MGMT promoter methylation

Two probes used by the MGMT-STP27 approach were not reliably covered in all analyzed samples. Methylation frequencies over all CpG sites covering the MGMT promoter region were averaged. Using pyrosequencing as gold standard, methylated and unmethylated samples were found to have a significant difference in their average methylation (Wilcoxon rank sum test p-value = 2.719e-06). As shown in FIG. 5, a threshold of 10% was assigned for MGMT promoter methylation status.

The embodiments of the present disclosure combine targeted nanopore sequencing and a classification algorithm. Both the classification algorithm and the nanopore sequencing system use the same target gene sites, i.e., the nanopore sequencing system sequences only the target gene sites required by the classification algorithm to classify the cancer. In other words, the nanopore sequencing system selectively sequences certain polymers in a pool while rejecting other polymers. Therefore, the cancer classification of the present disclosure is very flexible in target selection, runs efficiently on single samples, and can be initiated immediately upon receipt of frozen sections.

In addition, the targeted nanopore sequencing of the present disclosure analyzes the initial nucleotide sequence, i.e., nucleotide data rather than raw measurement signals, to determine whether the initial nucleotide sequence corresponds to the at least one target gene site. Using the nucleotide sequence rather than raw signals (e.g., ionic current signals) to match the initial nucleotide sequence to the at least one target gene site utilizes reasonable computational resources and provides enrichment of targets. Furthermore, since the embodiments of the present disclosure do not use raw signal comparison to determine whether the initial nucleotide sequence corresponds to the at least one target gene site, there is no need to convert the reference genomes, i.e., the at least one target gene site, into signal space.

While the foregoing is directed to embodiments of the disclosure, other and further embodiments of the disclosure may be devised without departing from the basic scope thereof, and the scope thereof is determined by the claims that follow.

## Claims

1. A computer-implemented method (100) for cancer diagnosis, the method comprising:
a) selectively sequencing polymers of a biological sample according to at least one target gene site by translocating the polymers through nanopores of a nanopore sequencing system, including:
(i) analyzing (120) an initial nucleotide sequence of a first polymer of the biological sample while the first polymer is translocating through a nanopore of the nanopore sequencing system to determine whether the initial nucleotide sequence corresponds to the at least one target gene site; and
(ii) continuing (150) the sequencing of the first polymer to obtain measurement data of the first polymer only if the initial nucleotide sequence of the first polymer corresponds to the at least one target gene site;
b) determining (160), based on the measurement data, a biological state of a nucleotide sequence of the first polymer corresponding to the at least one target gene site; and
c) classifying (170) a cancer using a classification algorithm based on the biological state of the nucleotide sequence of the first polymer, wherein the classification algorithm is trained based on the at least one target gene site and biological state data pertaining to cancer types.

2. The computer-implemented method (100) of claim 1, wherein the initial nucleotide sequence of the first polymer is a subset of the nucleotide sequence of the first polymer obtained when the first polymer has partially translocated through the nanopore.

3. The computer-implemented method (100) of claim 1 or 2, wherein step a) further includes:
rejecting (140) the first polymer if the initial nucleotide sequence of the first polymer does not correspond to the at least one target gene site.

4. The computer-implemented method of claim 3, wherein rejecting (140) the first polymer includes:
ejecting the first polymer from the nanopore; or
ceasing taking measurements from the nanopore.

5. The computer-implemented method (100) of claim 3 or 4, further including:
after the first polymer has been rejected, taking measurement from a second polymer translocating through the nanopore.

6. The computer-implemented method (100) of any one of claims 1 to 5, wherein step a) further includes:
obtaining raw measurement data from the nanopore; and
performing base-calling to obtain at least the initial nucleotide sequence of the first polymer.

7. The computer-implemented method (100) of claim 6, wherein the base-calling uses a neural network, in particular a recurrent neural network.

8. The computer-implemented method (100) of any one of claims 1 to 7, wherein selectively sequencing polymers of a biological sample according to at least one target gene site of step a) includes:
sequencing (i) the nucleotides of the first polymer corresponding to the at least one target gene site and (ii) a predetermined number of nucleotides upstream and/or downstream of the at least one target gene site.

9. The computer-implemented method (100) of any one of claims 1 to 8, wherein the biological state is selected from the group consisting of a mutation state and an epigenetic state of the nucleotide sequence of the first polymer.

10. The computer-implemented method (100) of claim 9, wherein the mutation state is a copy number variation and/or the epigenetic state is a methylation state.

11. The computer-implemented method (100) of claim 9 or 10, wherein the biological state of the nucleotide sequence of the first polymer is derived from the measurement data using one or more neural networks, in particular deep learning-based neural networks.

12. The computer-implemented method (100) of any one of claims 1 to 11, wherein the at least one target gene site includes at least one CpG position.

13. The computer-implemented method (100) of any one of claims 1 to 12, wherein the at least one target gene site is a plurality of target gene sites, and wherein a respective biological state is determined for each of the plurality of target gene sites.

14. A computer-readable storage medium having computer-executable instructions stored, that, when executed, cause a computer to perform a method (100) according to any one of claims 1 to 13.

15. A system for diagnosing cancer, comprising:
one or more processors; and
a memory coupled to the one or more processors and comprising instructions executable by the one or more processors to implement the method (100) according to any one of claims 1 to 13.
